# EUROPEAN PATENT APPLICATION

(11) **EP 1 287 813 A1**
(43) Date of publication of application: **05.03.2003**
(21) Application number: 02255985.0
(22) Date of filing: 28.08.2002
(51) Int. Cl.: A61K 7/32

(54) **Antiperspirant deodorant emulsion**

(30) Priority: 28.08.2001 US 941164
(71) Applicant: Coty Inc., New York, NY 10019 (US)
(72) Inventor: Patel, Amit, Passaic 07055, New Jersey (US); Schamper, Thomas, Cranbury 08512, New Jersey (US)
(74) Representative: Fairbairn, Angus Chisholm

(57) **Abstract**

The present invention includes an antiperspirant deodorant emulsion product comprising a spray or a roll-on or a wipe product. The antiperspirant decodorant emulsion includes a stable phase of oil and water, and an antiperspirant phasetand a fragrance phase. The stable phase of oil and water comprises two or more of glyceryl stearate, ceteareth-20, cetyl palmitate, cetearyl alcohol and ceteareth-12, dicaprylyl ether, coco-caprylate/caprate, steareth-2, PPG 15 stearyl ether and water.

## Description

### Background of the Invention

The present invention relates to antiperspirant deodorant emulsions and to a method for making antiperspirant deodorant emulsions.

Antiperspirant products have typically been available in forms such as a stick, a fluid, a roll-on, a gel, and a spray. Spray embodiments have included aerosols, natural sprays and squeeze sprays. In order to be successful in these forms, antiperspirant products have had to display stability and effectiveness over time, and have had to be capable of uniform application. It is desirable that antiperspirants also be gentle to skin. This goal is daunting to achicve because antiperspirant salts have typically been applied in high concentration and are astringent when applied to skin.

One difficulty encountered in making an antiperspirant emulsion is related to an inherent instability of the emulsions. Emulsions are thermodynamically unstable because of the disparate physical properties of components that make up the emulsion. Emulsions rely upon a kinetic hindrance to delay de-emulsification. This kinetic hindrance is overcome when emulsions are stored under conditions of elevated temperature and when emulsions arc subjected to cycles of high and low temperature. The process of de-emulsification reduces viscosity of the emulsions and ultimately results in a separation of aqueous and oil phases.

One other problem with antiperspirant deodorants is that they have tended to be sticky when applied. In some instances, stickiness has been reduced by forming a suspension of antiperspirant salts in a volatile silicone liquid. However, antiperspirant deodorant formulations that include these suspensions have tended to leave an undesirable white, powdery residue. In another formulation, U.S. Pat. No. 5,833,963, which issued November 10, 1998, describes an aqueous-based antiperspirant formulation free of emulsions or stringent alcohol additives.

Another formulation is described in U.S. Pat. No, 4,732,754, which issued March 22, 1988. The antiperspirant formulation includes an astringent material suspended in a hydrophobic medium to form an emulsion. The hydrophobic material includes stcaryl alcohol and cetyl alcohol, U.S. Pat. No. 4,803,195, which issued Feb. 7, 1989, describes a perfume base that is combined with a film-forming substrate and an emulsifying agent.

U.S. Pat. No. 6,221,345, which issued April 24, 2001, describes a formulation comprising antiperspirant in an emollient. The emollient included water and a humectant. The humectant included polyols and alcohols. The formulation also included a moisturizing cream. U.S. Pat. No. 5,942,216, which issued August 24, 1999, describes an addition of water to a water-in-oil emulsion to form water-in-oil-in-water multiple emulsions.

### Summary of the Invention

In its product aspect, the present invention includes a stable antiperspirant spray emulsion, a stable antiperspirant roll-on emulsion, and a stable antiperspirant wipe emulsion product. In one embodiment, the stable antiperspirant emulsion deodorant comprises a phase inversion temperature phase (PIT phase), an antiperspirant phase, and a fragrance phase. The phase inversion temperature phase comprises an oil phase and a water phase. The PIT phase comprises two or more of glyceryl stearate, ceteareth-20, cetyl palmitate, ceteryl alcohol and ceteareth-12, dicaprylyl ether, coco-caprylatc/caprate, steareth-2, PPG-15 stearyl ether, and water.

In another embodiment for a stable antiperspirant deodorant of the present invention, the antiperspirant deodorant comprises a phase inversion temperature phase. The phase inversion temperature phase comprises glyceryl stearate, ceteareth-20, cetyl palmitate, cetearyl alcohol, ceteareth-12, dicaprylyl ether, and coco-caprylate/caprate and a water phase.

In another embodiment, the present invention includes a stable antiperspirant deodorant emulsion that comprises steareth-2 and PPG-15 stearyl ether.

In its method aspect, the present invention includes a method for making a stable antiperspirant deodorant emulsion. The method includes providing water and heating the water to a temperature of 87 degrees Centigrade and maintaining the water temperature at 87 degrees Centigrade. The method also includes blending two or more ingredients of glyceryl stearate, ceteareth-20, cetyl palmitatc, cetearyl alcohol, ceteareth-12, dicaprylyl ether and coco-caprylate/caprate to form an oil phase. The oil phase is heated to 87 degrees Centigrade. Water is added to the oil phase in a manner effective to prevent air entrapment. The water is added to form a PIT emulsion.

In another embodiment, the present invention includes a method for making another stahle antiperspirant deodorant emulsion. The method includes mixing steareth-2 and PPG-15 stearyl ether to make an oil phase. The oil phase is heated at 70 to 73 degrees Centigrade and is maintained at 70 to 73 degrees Centigrade. A water phase comprising water, allantoin and steareth-20 is also prepared and heated to 73 to 77 degrees Centigrade. The water phase is added to the oil phase to form an emulsion. An antiperspirant phase is added to the emulsion to form the stable antiperspirant deodorant emulsion.

### Detailed Description

In its product aspect, the present invention comprises a stable antiperspirant deodorant spray emulsion and a stable antiperspirant deodorant roll-on emulsion and a stable antiperspirant emulsion applied in conjunction with a wipe applicator. The term, "spray emulsion" as used herein, refers to pump spray, squeeze spray, and pressurized aerosols, and other types of spray products.

The stable antiperspirant deodorant emulsions of the present invention comprise, in one embodiment, an oil phase that includes a mixture of glyceryl stearate, ceteareth-20, cetyl palmitate, cetearyl alcohol, ceteareth-12, dicaprylyl ether and coco-caprylute/caprate. The oil phase is blended with water to form a phase inversion temperature, FIT, emulsion. In one embodiment, the emulsion is blended with a second aqueous phase comprising water, glycerin, and allantoin to form a stahle emulsion. An antiperspirant phase that includes aluminum sesquichlorohydrate and water is prepared and is added to the stahle emulsion. A mixture of PEG 40 hydrogenated castor oil, and fragrance is prepared to form a fragrance phase. The fragrance phase is then added to the antiperspirant deodorant cmulsion. The antiperspirant deodorant emulsion embodiments of the present invention include water in a concentration within a range of 40% to 90%, w/w and in one embodiment, 71.274% by weight of the emulsion.

Another stable antiperspirant deodorant emulsion of the present invention includes a water phase comprising water, allantoin and steareth-20. This water phase is blended with an oil phase comprising steareth-2 and PPG-15 stearyl ether to form a stable emulsion. An antiperspirant phase; aluminum chlorohydrate and water, is added to a stable emulsion to form a stable antiperspirant emulsion. A fragrance phase is then added to the antiperspirant emulsion to form a stable, finished antiperspirant deodorant emulsion. The total water content in this stable cmulsion formulation is within a range of 40% to 90%, and, in one embodiment, is 70.90% by weight.

The antiperspirant deodorant stable spray emulsion embodiments of the present invention. when used as a spray product, form finely dispersed droplets of emollients that arc enclosed in a water droplet, when the spray cmulsion is atomized. Aqueous droplets include water and antiperspirant actives.

All of the antiperspirant deodorant spray emulsion and the antiperspirant deodorant roll-on emulsion and antiperspirant deodorant wipe emulsion product display a stability that has not heretofore been observed. The emulsions do not break over time and are resistant to breaking down under conditions of temperature extremes, particularly elevated temperature. Furthermore, the emulsions of the present invention display stability under conditions of repeated heating and cooling cycles. That the antiperspirant deodorant emulsions of the present invention display stability over time as well as over temperature elevation and variability is unexpected.

Antiperspirant deodorant emulsions have tended to be thermodynamically unstable. The emulsions have tended to de-emulsify in storage, especially at temperatures encountered in warmer climates. The emulsions have de-emulsified when subjected to temperatures that cycle between hot temperatures and cold temperatures. Antiperspirant salts in elevated concentrations have also de-emulsified emulsions. The antiperspirant deodorant emulsions of the present invention do not de-emulsify when subjected to temperature cycling or elevated temperatures or elevated concentrations of antiperspirant actives.

Furthermore, the roll-on emulsion embodiments and spray emulsion embodiments and wipe product embodiments provide effective antiperspirant protection while conditioning skin.

In some embodiments, the water used in the spray emulsion embodiments, wipe product embodiments, and roll-on emulsion embodiments of the present invention is de-ionized water and is present in a concentration of 40 to 60 percent by weight of the formulation. In one embodiment, the water is present in a concentration of 50.90 percent by weight of the formulation,

The oil phase is prepared substantially free of water. Trace amounts of water may he present in the components of the oil phase. For these embodiments, the glyceryl stearate, ceteareth-20, cetyl palmitate, cetearyl alcohol and ceteareth-12 arc obtainable from Cognis Corp. of Cincinnati, Ohio. This composition is known by the tradename Emulgade SE-PF or Emulgade SE. The concentration range is 1.50 to 6.50 percent. In one embodiment, the concentration is 4.50 percent. Emulgade is substantially free of water but may contain trace amounts.

The glyceryl stearate, cetyl palmitate, coco-caprylate/caprate and dicaprylyl ether act as skin conditioners and emollients. The cetearyl alcohol acts as an emulsion stabilizer and a viscosity increasing agent. The ceteareth-12, ceteareth-20 and steareth-20 act as surfactants and emulsifiers. The allantoin is an anti-irritant.

The ceteareth-20 is obtainable from Cognis Corp., and has a trade name, Eumulgin B-2 or Eumulgin B-2 flakes. The ceteareth-20 has a concentration of 0.15 to 2.0 percent of the phase. In one embodiment, the concentration is 0.90 percent. The dicaprylyl ether, known by tradename Cetiol OE, is obtainable from Cognis Corp. The dicaprylyl ether is present in a concentration of 2.0 to 8.0 percent. In one embodiment, the concentration was 5.00 percent by weight. The coco-caprylate/caprate has a tradename, Cetiol LC, which is obtainable from Cognis Corp. The Cetiol LC is present in a concentration of 2.0 to 8.0 percent and in one embodiment, is present in a concentration of 5.00 percent.

The allantoin, used for some embodiments, is available from Sutton Labs of Wayne, NJ, and is present in a concentration ranging from 0 to 0.30 percent. In one embodiment, the concentration is 0.1 percent, by weight. The steareth-20 has a Iradename, Brij 78, and is obtainable from Uniqucma of New Castle, DE. The steareth-20 is present in a concentration of 1.0 to 4.0 percent. In one embodiment, the concentration is 1.8 percent by weight.

In its method aspect, the present invention includes a method for making a stable antiperspirant deodorant spray emulsion, roll-on emulsion, and a wipe product emulsion. The spray embodiments include pump spray, squeeze spray, and pressurized aerosols. To make the stable antiperspirant deodorant emulsion, a water phase mixture of de-ionized water, is heated to a temperature of about 87 degrees Centigrade and is maintained at this temperature.

Ingredients of the oil phase are blended in a vessel, equipped with a mixer such as a sidesweep mixer. The ingredients are added one-at-a-time. The oil phase ingredients are blended to form a mixture. The mixture is heated to 85 to 86 degrees Centigrade with very slow mixing to avoid air entrapment.

Once the oil phase mixture is at 85 degrees Centigrade and is uniform and the de-ionized water is at a temperature of 87 degrees Centigrade, the water phase is added to the oil phase mixture very slowly to avoid air entrapment. Upon addition of the uncolored water to the uncolored oil phase mixture, taking care to avoid incorporation of air, a blue colored emulsion appears. The blue colored emulsion appears after about 10 to 15 minutes, provided the phases are uniform and provided no coloring agents are included in either the water phase or the oil phase. This blue color identifies the phase inversion temperature, PIT, emulsion. After the blue color is observed, mixing is continued, slowly, until the PIT emulsion is cooled. It is very important that the water is added to the waxy mixture very slowly, to avoid aeration.

A second aqueous phase that includes water, glycerin, and allantoin is next added to the PIT emulsion. The water in the second aqueous phase is de-ionized and is present in a concentration within a range of 5.0 to 50.0 percent by weight. In one embodiment, the water concentration is 26.25 percent by weight. The glycerin, obtainable from Ashland Chemical Co. of Covingron, KY, is present in a concentration of 0,25 to 5.0 percent by weight. In one embodiment, the concentration is 1.5 percent by weight, The allantoin concentration range is 0 to 0.30 percent by weight. In one embodiment, the concentration range is 0.10 percent.

The ingredients of the second aqueous phase are added to a vessel equipped with a Lighnin mixer or other similar mixer. In one embodiment, the ingredients are premixed prior to addition to the vessel. The ingredients of the second aqueous phase are completely and uniformly dissolved. The second aqueous phase is then added to the mixture in a manner that retains uniformity of the emulsion and that causes minimum air entrapment.

The ingredients, aluminum sesquichlorohydrate and water, are blended to form the antiperspirant phase. In one embodiment, a commercial product having the tradename of Rcach 301 Solution is employed. The Reach 301 Solution is obtainable from Rchcis of Berkley Heights, NJ. This component is added in a concentration of 5.0 to 40.0 percent and in one embodiment, is added in a concentration of 20.0 percent. While aluminum sesquichlorohydrate is described, it is believed that other antiperspirant actives are suitable for use in the antiperspirant emulsion deodorant of the present invention. The other antiperspirant actives include compounds or compositions that have antiperspirant activity, such as astringent metallic salts. These salts include inorganic and organic salts of aluminum, zirconium, and zinc and mixtures thereof. Specific examples include aluminum halides, aluminum hydroxide halides, zirconyl oxide halides, zirconyl hydroxy halides and mixtures of these materials, such as aluminum chloride, aluminum chlorohydrex PEG, aluminum chlorohydratc, aluminum chlorohydrex PG, aluminum dichlorohydrex PG, aluminum sesquichlorohydrex PEG, aluiminum sesquichlorohydrex PG, aluminum zirconium octachlorohydrate, aluminum zirconium octachloroydrex GLY, aluminum zirconium pentachlorohydrate, aluminum zirconium octachloroydrex GLY, aluminum zirconium tetrachlorohydrate, aluminum zirconium tetrachlorohydrex GLY, aluminum zirconium trichlorohydrate, aluminum zirconium trichlorohydrex GLY, and mixtures thereof. The antiperspirant salts are solubilized in water.

Once the ingredients of the antiperspirant phase are blended to form a solution, the solution is added to the PIT emulsion at 55 degrees Centigrade with slow mixing. It is important to avoid aeration.

The fragrance phase includes PEG 40 hydrogenated castor oil and fragrance. The PEG-40 hydrogenated castor oil is available under the trade name, Cremophor RH 40, manufactured by BASF. The Cremophor RH 40 concentration ranges from 0.10 to 2.0 percent. In one embodiment, the concentration is 0.75 percent. The fragrance concentration ranges from 0.05 to 4.0 percent. In one embodiment, the concentration is 1.00 percent.

While a fragrance is described, other active materials may be used in the formulation of the present invention. The other deodorant active materials include ammonium phenol sulfonate, benzalkonium chloride, benzethonium chloride, bromochlorophene, cetylpyridinium chloride, 20 chlorophyllin-copper complex, chlorothymol, chloroxylenol, cloflucarban, dequalinium chloride, dichlorophene, dichloro-m-xylenol, hexachlorophene, lauryl pyridinium chloride, methylbenzethonium chloride, phenol, triclocarbone, triclosan, and mixtures thereof.

Ingredients of the fragrance phase are premixed and then added to a vessel equipped with a Lighnin mixer or a similar mixer. The ingredients are mixed until they are completely dissolved and uniform. The final phase is added to the PIT emulsion at 42 degrees Centigrade with slow mixing in order to avoid incorporation of air.

The PIT emulsion with all of the phases incorporated is maintained with moderate agitation, and cooled to 30 degrees Centigrade to form the stable antiperspirant deodorant emulsion. When the emulsion is at 30 degrees Centigrade, a sample of the emulsion is tested. When the emulsion is approved, the emulsion is transferred to properly labeled containers for antiperspirant products. In one embodiment, the emulsion is transferred to containers equipped with spray nozzles that deliver the antiperspirant emulsion in aerosol form. The container may be a pump spray container, a squeeze spray container or a pressurized aerosol container. It is believed that other containers that dispense the antiperspirant emulsion as an aerosol are suitable for use in the present invention.

A summary of ingredients in each phase, for one antiperspirant deodorant emulsion is summarized in the tables described herein:

| **Chemical** | **Raw Material %Range w/w** | **Phase** |
|---|---|---|
| Water | 10.0-50.0 | Water |
| Emulgade SE | 1.5-6.5 | Oil |
| Ceteareth-20 | 0.15-2.0 | Oil |
| Coco-caprylate/caprate | 2.0-8.0 | Oil |
| Water | 5.0-50.0 | Second Aqueous Phase |
| Glycerin | 0.25-5.0 | Second Aqueous Phase |
| Allantoin | 0.0-0.30 | Second Aqueous Phase |
| Aluminum Sesquichchlorohydrate & Water | 5.0-40.0 | Antiperspirant |
| PEG-40 Hydrogenated castor oil | 0.10-2.0 | Fragrance |
| Fragrance | 0.05-4.0 | Fragrance |

While a spray receptacle is described, it is believed that the antiperspirant deodorant emulsion may be dispensed in a wipe. The wipe may have a cellulosic structure, such as a tissue, a non-woven structure, foam or a combination thereof that has a one-ply or that has a multi-ply structure. The antiperspirant deodorant is applied between two of the multi- plies or on a surface of a wipe with one ply or multi-plies. Suitable wipe substrates include conventional homogeneous paper, through-air-dried paper, a differential-density paper, or a differential-basis weight paper or foam.

In another embodiment, a stable antiperspirant deodorant roll-on emulsion is prepared. This method is also usable to prepare some spray embodiments and wipe product embodiments. The roll-on emulsion includes an oil phase that is prepared by blending steareth-2 and PPG 15 stearyl ether. The steareth-2, known as Brij 72, is available form Uniqcma of New Castle, DE. The Brij 72 is present in a concentration of 1.0 to 6.0% by weight, and, for one embodiment, is present in a concentration of 3.00 percent by weight. The PPG-15 stearyl ether has a trade name, Arlamol E, and is obtainable from Uniqema. The Arlamol E is present in a concentration of 1.0 to 6.0 percent by weight, and, in one embodiment, is present in a concentration of 3.00 percent by weight. The oil phase is healed to 70 to 73 degrees Centigrade and is maintained at 73 degrees Centigrade.

A water phase that includes de-ionized water in a concentration of 40.0 to 60.0% by weight, and, in one embodiment, 50.90% by weight is prepared. The water phase also includes allantoin in a concentration of 0.10% by weight. The water phase also includes steareth-20 in a concentration of 1.8% by weight. The water phase is heated to 73 to 77 degrees Centigrade. The water phase is added to the oil phase to form an emulsion.

This embodiment includes an antiperspirant phase. The antiperspirant phase includes aluminum chlorohydrate and water. The aluminum chlorohydrate and water is available under the trade name, Chlorhydrol 50% Solution, and is available from Reheis. The Chlorhydrol 50% solution is added in a concentration of 5.0 to 55.0%, and, in one embodiment, is present in a concentration of 40.00 percent by weight.

A fragrance is added in a concentration range of 0.05 percent to 4.0 percent. In one embodiment, the fragrance is added in a concentration of 1.20 percent by weight.

A summary for a second antiperspirant deodorant roll-on emulsion is as follows:

| | | |
|---|---|---|
| Stcarcth-2 | 1.0-6.0 | Oil |
| PPG 15 Stearyl ether | 1.0-6.0 | Oil |
| Water | 40.0-60.0 | Water |
| Allantoin | 0.0-0.30 | Water |
| Steareth-20 | 1.0-4.0 | Water |
| Fragrance | 0.05-4.0 | Fragrance |
| Aluminum Chlorohydrate & Water | 5.0-55.0 | Antiperspirant |

To make this stable antiperspirant deodorant roll-on embodiment, the ingredients of the oil phase and the water phase arc separately blended in vessels equipped with mixers such as a sidesweep mixer. The oil phase is heated to 70 to 73 degrees Centigrade and the water phase is heated to 73 to 77 degrees Centigrade. The phases are blended with slow addition of the water phase to the oil phase in order to avoid air entrapment to form an emulsion phase. The emulsion phase, at ambient temperature, is slowly added to the emulsion, in a manner that avoids aeration. The fragrance is added once the emulsion with antiperspirant is cooled to about 42 degrees Centigrade.

When the emulsion is at 30 degrees Centigrade, a sample of the emulsion is tested. When the emulsion is approved, the emulsion is transferred to properly labeled containers for roll-on antiperspirant deodorant products. For some embodiments, this emulsion is applied to wipes, such as are described above.

For some embodiments, the antiperspirant deodorant emulsion additionally includes preservatives, vitamins, materials that absorb UV radiation, antioxidants, enzymes, colors, and coenzymes.

The examples described below are presented to describe specific embodiments of the present invention and are not presented to limit the present invention.

### Example 1

An antiperspirant spray emulsion was prepared with the phases described herein:

| **Ingredients** | **Concentration** | **Phase** |
|---|---|---|
| Water | 35.00 | Water |
| Emulgade SE | 4.50 | Oil |
| Ceteareth-20 | 0.90 | Oil |
| Dicaprylyl Ether | 5.00 | Oil |
| Coco-Caprylate/.Caprate | 5.00 | Oil |
| Water | 26.25 | Second Aqueous Phase |
| Glycerin | 1.50 | Second Aqueous Phase |
| Allantoin | 0.10 | Second Aqueous Phase |
| Reach 301 Solution | 20.00 | Antiperspirant |
| PEG-40-hydrogenated castor oil | 0.75 | Fragrance |
| Fragrance | 1.00 | Fragrance |
| | 100.00 | |

### Example 2

An antiperspirant roll-on emulsion was prepared from the formulation described herein.

| **Ingredients** | **Concentration** | **Phase** |
|---|---|---|
| Stcarcth-2 | 3.00 | Oil |
| PPG 15 Stearyl Ether | 3.00 | Oil |
| Water | 50.90 | Water |
| Allantoin | 0.10 | Water |
| Steareth-20 | 1.80 | Water |
| Aluminum Chlorohydrate & Water | 40.00 | Antiperspirant |
| Fragrance | 1.2 | Fragrance |
| Total | 100 | |

## Claims

1. An antiperspirant deodorant emulsion product, comprising:
a phase inversion temperature phase, comprising:
an oil phase comprising two or more of a mixture of glyceryl stearate, ceteareth-20, cetyl palmitate, cetearyl alcohol and ceteareth-12, dicaprylyl ether, coco-caprylate/caprate, steareth-2, PPG 15 stearyl ether, and water; and
a water phase, wherein the combination of the oil phase and the water phase forms a phase inversion temperature phase; and
an antiperspirant.

2. The antiperspirant deodorant emulsion product of claim 1 wherein the phase inversion temperature phase is blue in an absence of a coloring agent.

3. The antiperspirant deodorant emulsion product of claim 1 and further comprising a receptacle for containing the antiperspirant deodorant emulsion.

4. The antiperspirant deodorant emulsion of claim 1 wherein the oil phase comprises glyceryl stearate, ceteareth-20, cetyl palmitate, cetearyl alcohol, ceteareth-12, and dicaprylyl ether.

5. The antiperspirant deodorant emulsion of claim 1, further comprising a fragrance phase.

6. An antiperspirant deodorant emulsion, comprising:
a phase inversion temperature phase, comprising: glyceryl stearate, ceteareth-20, cetyl palmitate, cetearyl alcohol, ceteareth-12, dicaprylyl ether, and coco-caprylate/caprate; and
an anti-perspirant.

7. An antiperspirant roll-on deodorant, comprising:
steareth-2, PPG 15 stearyl ether and an antiperspirant.

8. The antiperspirant deodorant of claim 1 wherein the antiperspirant comprises aluminum chlorohydrate.

9. The antiperspirant deodorant of claim 7 wherein the antiperspirant comprises aluminum sesquichlurohydrate.

10. The antiperspirant deodorant of claim 3 wherein the receptacle comprises a mechanism for releasing the emulsion as a spray.

11. The antiperspirant deodorant of claim 3 wherein the receptacle comprises a mechanism for releasing the emulsion as a roll-on.

12. The antiperspirant deodorant of claim 3 wherein the receptacle releases the emulsion from a wipe.

13. A method for making an antiperspirant deodorant, comprising:
providing water and heating the water to a temperature of 87 degrees Centigrade and maintaining the water temperature at 87 degrees Centigrade;
blending two or more ingredients of glyceryl stearate, ceteareth-20, cetyl palmitale, cetearyl alcohol, ceteareth-12, dicaprylyl ether and coco-caprylate/caprate, one-at-a-time, to form an oil phase arid heating the oil phase to 87 degrees; and
adding the water to the oil phase in a manner effective for preventing air entrapment to form a stable emulsion.

14. The method of claim 13 and further comprising mixing the stable emulsion until a blue color is observed in an absence of a coloring agent.

15. The method of claim 13 and further comprising mixing the stable emulsion until the stahle emulsion is cooled.

16. The method of claim 13 and further comprising preparing a second aqueous phase by mixing two or more of glycerin, water and allantoin in a manner effective to prevent air entrapment.

17. The method of claim 16 and further comprising adding the second aqueous phase to the stable emulsion.

18. The method of claim 17 and further providing an antiperspirant.

19. The method of claim 18 and further comprising cooling the stable emulsion to 55 degrees Centigrade and adding the antiperspirant to the stable emulsion in a manner effective to avoid aeration.

20. The method of claim 19 and further comprising cooling the stable emulsion with the antiperspirant to 42 degrees Centigrade and adding the second aqueous phase to the cooled stable emulsion.

21. A method for making a stable antiperspirant emulsion. comprising:
providing an oil phase comprising steareth-2 and PPG-15 stearyl ether;
heating the oil phase to about 70 to 73 degrees Centigrade;
providing a water phase and heating the water phase to 73 to 77 degrees Centigrade;
adding the water phase to the oil phase to form an emulsion; and
adding an antiperspirant to the emulsion to form a stable antiperspirant emulsion.
